# EUROPEAN PATENT SPECIFICATION

(11) **EP 3 760 198 B1**
(45) Date of publication and mention of the grant of the patent: **06.09.2023**
(21) Application number: 19761141.1
(22) Date of filing: 19.02.2019
(51) Int. Cl.: A61K 9/06, A61K 9/107, A61K 47/12, A61K 47/26, A61K 47/38, A61K 47/22, A61K 47/18, A61K 31/196, A61K 47/10

(54) **DICLOFENAC-CONTAINING EMULSIFIED GEL COMPOSITION**
DICLOFENAC-ENTHALTENDE EMULGIERTE GELZUSAMMENSETZUNG
COMPOSITION DE GEL ÉMULSIFIÉE CONTENANT DU DICLOFÉNAC

(30) Priority: 27.02.2018 JP 2018033220
(43) Date of publication of application: 06.01.2021
(73) Proprietor: Hisamitsu Pharmaceutical Co., Inc., Tosu-shi, Saga 841-0017 (JP)
(72) Inventor: NAKANISHI Toshihiro, Tosu-shi, Saga 841-0017 (JP); NAGASE Yuko, Tosu-shi, Saga 841-0017 (JP); MATSUMURA Shinya, Tosu-shi, Saga 841-0017 (JP); KOSE Yasuhisa, Tosu-shi, Saga 841-0017 (JP)
(74) Representative: Grünecker Patent- und Rechtsanwälte PartG mbB
(86) International application number: PCT/JP2019/006127
(87) International publication number: WO 2019/167728

(56) References cited:
- WO-A1-2016/038553
- JP-A- H0 912 452
- JP-A- H0 912 452
- JP-A- 2005 047 908
- US-A1- 2004 101 538
- US-A1- 2010 099 766

## Description

### Technical Field

The present invention relates to a diclofenac-containing emulsified gel composition.

### Background Art

A gel is known as a type of dosage forms of transdermal formulations, and in particular, an emulsified gel is excellent in use feeling at the time of and after application onto the skin as compared with a gel not emulsified. Besides, an emulsified gel comprises an aqueous component and an oily component, and the oily component is stably dispersed in the gel.

Diclofenac is known as a nonsteroidal anti-inflammatory agent, and is widely used in chemotherapy. Besides, a diclofenac-containing gel is also known (Patent Literatures 1 to 3). It is known that, in a stability test of a diclofenac-containing formulation, 1-(2,6-dichlorophenyl)-2-indolinone is generated under heating condition.

### Citation List

### Patent Literature

Patent Literature 1: JP 2015-522049 T
Patent Literature 2: JP 2016-193862 A
Patent Literature 3: JP 2017-81901 A

Further prior art relating to gel compositions is disclosed in JP H09 12452 A, US 2004/101538 A1, WO 2016/038553 A1 and US 2010/099766 A1.

### Summary of Invention

### Technical Problem

The present inventors have, however, found that drug stability of diclofenac sodium is low in an emulsified gel comprising diclofenac sodium in some cases.

Therefore, an object of the present invention is to provide a diclofenac-containing emulsified gel composition excellent in drug stability, according to claim 1.

### Solution to Problem

The present invention provides the following:
An emulsified gel composition comprising diclofenac sodium, water, a gelling agent, an antioxidant, and a surfactant having an HLB of 14 or more, in which the gelling agent is a nonionic water-soluble polymer selected from the group consisting of hydroxypropyl cellulose and hydrophobically-modified hydroxypropyl methyl cellulose, and
wherein the composition does not comprise an ionic polymer of comprises an ionic polymer in an amount of 0.5% by mass or less based on the mass of the whole emulsified gel composition.

Preferably, the nonionic water-soluble polymer comprises hydrophobically-modified hydroxypropyl methyl cellulose.

Preferably, the antioxidant comprises at least one compound selected from the group consisting of 2-mercaptobenzimidazole, disodium edetate, tocopherol, dibutyl hydroxytoluene, and propyl gallate.

Preferably, the surfactant having an HLB of 14 or more comprises at least one compound selected from the group consisting of polyoxyethylene sorbitan monococonut fatty acid ester, polyethylene glycol monostearate, polyoxyethylene phytosterol, polyoxyethylene cetyl ether, polyoxyethylene behenyl ether, and polyoxyethylene oleyl ether.

Preferably, a pH of the emulsified gel composition is 7.0 or more.

### Advantageous Effects of Invention

According to the present invention, a diclofenac-containing emulsified gel composition excellent in drug stability of diclofenac sodium can be provided. Besides, according to the present invention, use feeling at the time of or after application onto the skin is also excellent.

### Brief Description of Drawings

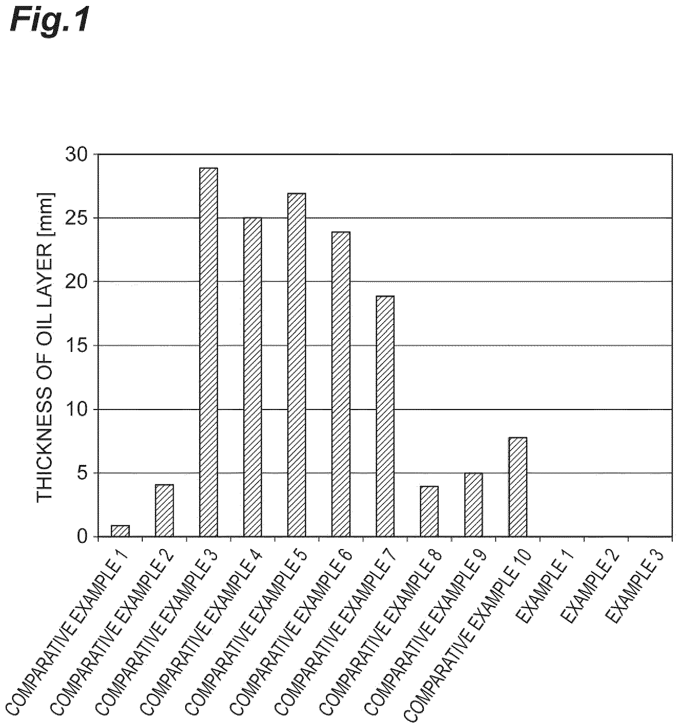

[Figure 1] Figure 1 is a graph illustrating results of Test 3.

### Description of Embodiments

The present invention will now be described in detail.

Herein, diclofenac or a salt thereof may be sometimes referred to as the "drug".

Herein, the term "emulsified gel composition" means a gel composition containing an aqueous phase (dispersion medium) and an oil phase (dispersoid). The aqueous phase refers to a continuous phase containing water and a water-soluble component such as a gelling agent, and the oil phase contains a hydrophobically-modified component or a lipophilic component, and is dispersed in the aqueous phase. The oil phase may be an oil droplet, and may be dispersed in the form of a micelle or a vesicle formed by a surfactant or the like. The aqueous phase and the oil phase are involved in improvement of characteristics such as use feeling of the emulsified gel composition.

Herein, the term "excellent drug stability" means that diclofenac or a salt thereof does not likely to generate a decomposition product in the emulsified gel composition even after long-term storage. The term "excellent drug stability" means that after storing the emulsified gel composition at 60°C for 1 month, a residual amount of the drug is 98% or more as compared with the amount before the storage.

Diclofenac sodium is also designated as sodium 2-[(2,6-dichlorophenyl)amino]phenylacetate, and is a nonsteroidal anti-inflammatory analgesic inhibiting cyclooxygenase. Besides, diclofenac sodium is effective for treatment of diseases or symptoms such as osteoarthrosis, periarthritis scapulohumeralis, tendonitis/tenosynovitis, peritendinitis, humeral epicondylitis, muscle pain (such as myofascial lumbago), and posttraumatic swelling/pain.

Diclofenac is known to easily generate various decomposition products through an intramolecular cyclic amidation reaction and an esterification reaction. Examples of the decomposition products of diclofenac include 1-[2,6-dichlorophenyl]-2-indolinone (molecular weight: 278.13), and ethyl 2-[(2,6-dichlorophenyl)amino]phenylacetate (molecular weight: 324.20).

The content of diclofenac sodium may be 0.1 to 3% by mass, and is preferably 0.5 to 1.5% by mass, and more preferably 0.8 to 1.2% by mass based on the mass of the whole emulsified gel composition. Diclofenac sodium may be comprised in the aqueous phase or may be comprised in the oil phase in the emulsified gel composition.

The water may be non-purified water, and is preferably purified water such as ion exchanged water, distilled water, or ultrafiltration water. The content of the water may be 20 to 60% by mass, and is preferably 30 to 50% by mass, and more preferably 35 to 45% by mass based on the mass of the whole emulsified gel composition. When the content of the water is 20% by mass or more, the resultant emulsified gel composition attains appropriate liquidity, and hence can be easily applied on the skin, and in addition, the applied skin surface is less sticky after the application. Besides, when the content of the water is 60% by mass or less, the gel composition applied onto the skin is more difficult to flow down.

The gelling agent is a nonionic water-soluble polymer selected from the group consisting of hydroxypropyl cellulose and hydrophobically-modified hydroxypropyl methyl cellulose.

The hydrophobically-modified cellulose derivative refers to a derivative of cellulose obtained by introducing a hydrophobically-modified group into a hydroxy group of cellulose or semi-synthetic cellulose. The hydrophobically-modified group may be an alkyl group having 4 to 30 carbon atoms, and may be an alkyl group having 12 to 24 carbon atoms such as a cetyl group, a lauryl group, a stearyl group, or an oleyl group. Besides, the hydrophobically-modified group may arbitrarily have an ether bond or a hydroxy group. Examples of the hydrophobically-modified cellulose derivative include hydrophobically-modified hydroxypropyl methyl cellulose (hydrophobically-modified HPMC), and hydrophobically-modified hydroxyethyl cellulose (HEC). Specifically, the "hydrophobically-modified HPMC" refers to HPMC into which a small amount of hydrophobically-modified group has been introduced. The hydrophobically-modified cellulose derivative is more excellent in thickening effect than a cellulose derivative such as HEC or HPC, and excellent also in compatibility with alcohol. The hydrophobically-modified cellulose derivative easily forms a thixotropic gel, is more excellent in shape retention, and can further inhibit stickiness otherwise caused after the application.

The hydrophobically-modified HPMC may comprise 0 to 33% by mass of a methoxy group, preferably comprises 10 to 30% by mass of a methoxy group, more preferably comprises 21.5 to 30% by mass of a methoxy group, and further preferably comprises 21.5 to 24% by mass or 27 to 30% by mass of a methoxy group based on the mass thereof. The hydrophobically-modified HPMC may contain 0 to 20% by mass of a hydroxypropyloxy group, preferably comprises 4 to 15% by mass of a hydroxypropyloxy group, and more preferably comprises 7 to 11% by mass of a hydroxypropyloxy group based on the mass thereof. The hydrophobically-modified HPMC may be HPMC having a stearyloxy group (stearyloxy HPMC). The stearyloxy HPMC may comprise 0.3 to 4.5% by mass of a stearyloxy hydroxypropyloxy group, preferably comprises 0.3 to 2% by mass of a stearyloxy hydroxypropyloxy group, and more preferably comprises 0.3 to 0.6% by mass or 1 to 2% by mass of a stearyloxy hydroxypropyloxy group based on the mass thereof. As the hydrophobically-modified HPMC, for example, SANGELOSE 60L, 60M, 90L, and 90M (all trade names, manufactured by Daido Chemical Corporation) may be used.

The gelling agent comprises a gelling agent having a cellulose structure (such as a β1,4-glycoside bond), comprises one or more compounds selected from the group consisting of HPC and hydrophobically-modified HPMC. When HPC or hydrophobically-modified HPMC is comprised, the viscosity of the resultant gel composition can be easily improved, so as to more easily inhibit the gel composition from falling off from the skin. Besides, spreadability in applying the gel composition onto the skin is further improved, and stickiness otherwise caused on the applied skin surface after rubbing the gel composition can be further inhibited.

A content of the gelling agent may be 0.5 to 5% by mass, is preferably 0.8 to 4% by mass, and more preferably 1.1 to 3% by mass based on the mass of the whole emulsified gel composition.

It is most preferable that the emulsified gel composition of the present embodiment does not comprise an ionic polymer. The emulsified gel composition may comprise, however, an ionic polymer such as a carboxyvinyl polymer, carboxymethyl cellulose, polyacrylic acid and a salt of any of these in an amount of 0.5% by mass or less based on the mass of the whole emulsified gel, such that the effects of the present invention are not impaired. When the emulsified gel composition comprises an ionic polymer, a content of the ionic polymer is 0.5% by mass or less based on the mass of the whole emulsified gel composition. When the emulsified gel composition comprises a carboxyvinyl polymer, interaction with a specific physiologically active substance such as diclofenac sodium is caused, and hence, the ionic polymer is easily aggregated, and in addition, crumbs are easily formed in rubbing the emulsified gel composition into the skin. The term "crumbs" means that when a composition is rubbed into the skin, a solid content contained in the composition is dried to form a grime-like (pasty) lump. As the content of the ionic polymer decreases, the resultant emulsified gel composition exhibits better effects. When the emulsified gel composition comprises HPC or hydrophobically-modified HPMC, the aggregation as described above is not caused, the viscosity can be appropriately increased, and in the application onto the skin, the gel composition can be inhibited from falling down and forming crumbs. Besides, the emulsified gel composition comprising hydrophobically-modified HPMC is excellent also in spreadability in application, and tends to be less sticky after the application.

The antioxidant may be a component capable of inhibiting autoxidation of diclofenac sodium with light or oxygen. Examples of the antioxidant include 2-mercaptobenzimidazole, disodium edetate, sodium hydrogen sulfite, sodium sulfite, sodium pyrosulfite, tocopherol, tocopherol acetate, dibutyl hydroxytoluene, dibutyl hydroxyanisole, ethyl gallate, propyl gallate, isopropyl gallate, oxybenzone, ascorbic acid, and ascorbyl palmitate. Dibutyl hydroxytoluene is preferred because sufficient antioxidant effect can be exhibited even when a content is low.

The content of the antioxidant may be 0.01 to 2% by mass, and is preferably 0.02 to 1% by mass, and more preferably 0.05 to 0.5% by mass based on the mass of the whole emulsified gel composition. The antioxidant may be comprised in the aqueous phase, or may be comprised in the oil phase in the emulsified gel composition.

The surfactant is a nonionic surfactant having an HLB value of 14 or more. An HLB value refers to a numerical value corresponding to hydrophilic-lipophilic balance of a surfactant, and is defined in a range of 0 to 20. An HLB value closer to 0 (zero) means stronger lipophilicity, and an HLB value closer to 20 means stronger hydrophilicity. For example, polyethylene glycol monolaurate (10 E. O.) (NIKKOL MYL-10, manufactured by Nikko Chemicals Co., Ltd.) has an HLB value of 12.5, and POE (15) cetyl ether (NIKKOL BC-15, manufactured by Nikko Chemicals Co., Ltd.) has an HLB value of 15.5.

Examples of such a surfactant include POE (9) lauryl ether, POE (21) lauryl ether, POE (25) lauryl ether, POE (10) oleyl ether, POE (15) oleyl ether, POE (20) oleyl ether, POE (50) oleyl ether, POE (12) secondary alkyl ether, POE lanolin alcohol, POE (30) lanolin, POE (15) cetyl ether, POE (20) cetyl ether, POE (23) cetyl ether, POE (25) cetyl ether, POE (30) cetyl ether, POE (40) cetyl ether, POE (20) stearyl ether, POE (20) behenyl ether, POE (30) behenyl ether, POE (20) POP (4) cetyl ether, polyethylene glycol monostearate (25 E. O.), polyethylene glycol monostearate (40 E. O.), polyethylene glycol monostearate (45 E. O.), polyethylene glycol monostearate (55 E. O.), polyethylene glycol-150 distearate, POE (60) sorbit tetraoleate, POE (20) sorbitan monostearate, POE (20) sorbitan monoisostearate, POE (20) sorbitan monooleate, POE (6) sorbit monolaurate, POE (20) sorbitan monococonut fatty acid ester, POE (30) cholestanol, POE (60) hydrogenated castor oil, POE (80) hydrogenated castor oil, POE (100) hydrogenated castor oil, decaglyceryl monomyristate, hexaglyceryl monolaurate, decaglyceryl monolaurate, POE (25) phytostanol, POE (20) phytosterol, and sodium di-POE (10) lauryl ether phosphate. Specifically, NIKKOL BL-9, NIKKOL BL-21, NIKKOL BL-25, NIKKOL BO-10V, NIKKOL BO-15V, NIKKOL BO-20V, NIKKOL BO-50V, NIKKOL BT-12, NIKKOL BWA-10, NIKKOL TW-30, NIKKOL BC-15, NIKKOL BC-20, NIKKOL BC-23, NIKKOL BC-25, NIKKOL BC-30, NIKKOL BC-40, NIKKOL BS-20, NIKKOL BB-20, NIKKOL BB-30, NIKKOL BPC-34, NIKKOL MYS-25V, NIKKOL MYS-40V, NIKKOL MYS-45V, NIKKOL MYS-55V, NIKKOL CDS-6000P, NIKKOL GO-460V, NIKKOL TS-10V, NIKKOL TI-10V, NIKKOL TO-10V, NIKKOL GL-1, NIKKOL TL-10, NIKKOL DHC-30, NIKKOL HCO-60, NIKKOL HCO-80, NIKKOL HCO-100, NIKKOL Decaglyn 1-M, NIKKOL Hexaglyn 1-L, NIKKOL Decaglyn 1-L, NIKKOL BPSH-25, NIKKOL BPS-20, and NIKKOL DLP-10 (all trade names, manufactured by Nikko Chemicals Co., Ltd.) may be used as the surfactant. In the above description, a numerical value described in parentheses following POE refers to an average number of moles of an oxyethylene unit added.

When the surfactant has a polyoxyethylene group, the HLB value can be changed in accordance with an average number of moles of an oxyethylene unit added. For example, an average number of moles of an oxyethylene unit added in a polyoxyethylene alkyl ether may be 20 to 150, and is preferably 30 to 150. POE sorbitan coconut fatty acid ester more preferably has an average number of moles of an oxyethylene unit added of 20 to 60.

Besides, an alkyl group of the polyoxyethylene alkyl ether is preferably an alkyl group having 10 to 24 carbon atoms, more preferably has 16 to 24 carbon atoms, and further preferably has 18 to 22 carbon atoms. A preferable surfactant is POE stearyl ether, POE oleyl ether, POE lauryl ether, POE myristyl ether, POE palmityl ether, POE octyldodecyl ether, POE cetyl ether, or POE behenyl ether. POE stearyl ether more preferably has an average number of moles of an oxyethylene unit added of 45 to 60. POE cetyl ether more preferably has an average number of molecules of an oxyethylene unit added of 23 to 60. POE behenyl ether more preferably has a number of moles of an oxyethylene unit added of 20 to 60.

Measurement of an HLB value may be performed by a method known to those skilled in the art. The measurement of an HLB value is performed by, for example, a method in which a surfactant having a known HLB used as a standard and a sample having an unknown HLB are respectively used to emulsify an oil used as a standard (such as liquid paraffin) and purified water, and a combination ratio at which the most stable emulsion can be obtained is compared between the standard and the sample. A composition used in the measurement may have a composition containing 40% by mass of an oil phase, 56% by mass of purified water, and 4% by mass of a surfactant or a sample having an unknown HLB. Besides, the measurement method may be determined by referring to description of JP 2010-099017 A, JP 2005-272750 A, JP 2002-301352 A and the like.

The content of the surfactant may be 0.5 to 4% by mass, is preferably 1 to 3% by mass, and more preferably 1.5 to 2.5% by mass based on the mass of the whole emulsified gel composition. When the content of the surfactant is 4% by mass or less, the application feeling is more excellent, and stickiness is minimally felt. When the content of the surfactant is 0.5% by mass or more, crumbs can be more sufficiently inhibited, and a more sufficient emulsified state is obtained.

The emulsified gel composition of the present embodiment may further comprise a lower alcohol. The lower alcohol functions as a liquid medium forming a gel together with the water-soluble polymer in the gel base. Besides, when the lower alcohol is comprised, a drying time after the application of the composition can be shortened, and the use feeling is further improved. The lower alcohol may be an aliphatic alcohol having 1 to 6 carbon atoms, and is preferably an aliphatic alcohol having 1 to 3 carbon atoms. As the alcohol has a larger number of carbon atoms, the drying takes longer time after the application. Examples of the lower alcohol include ethanol and isopropanol, and ethanol is preferred. These lower alcohols may be used singly or in combinations of two or more.

The content of the lower alcohol is not especially limited, and may be, based on the mass of the water comprised in the emulsified gel composition, 0.5 to 2-fold amount, is preferably 0.6 to 1.5-fold amount, and more preferably 0.8 to 1.2-fold amount. When the content of the lower alcohol is 0.5-fold or larger amount of the content of the water, the application feeling can be further improved, and the drying time after the application can be further shortened. When the content of the lower alcohol is 2-fold or smaller amount of the content of the water, swelling or dissolution of the gelling agent can be further accelerated, there is a tendency that the emulsified state of the gel composition can be easily retained, and stimulus to the skin by the alcohol can be further reduced.

The emulsified gel composition of the present embodiment may further comprise an optional component such as a second active ingredient, an oily component, an absorption enhancer, a dissolving agent, and a pH regulator.

In the emulsified gel composition of the present embodiment, hydrophobically-modified components (such as a specific physiologically active substance, an antioxidant, and an oily component) integrally form the oil phase, and the resultant is emulsified if necessary to obtain an emulsified gel.

The second active ingredient is not especially limited as long as it is a drug known to have pharmacological action in the medical field, and is a component excluding diclofenac and a salt thereof. Examples of the second active ingredient include anti-inflammatory analgesics (such as indomethacin, ketoprofen, felbinac, flurbiprofen, loxoprofen, ibuprofen, ibuprofen piconol, guaiazulene, allantoin, piroxicam, glycyrrhizic acid, glycyrrhetinic acid, salicylic acid, methyl salicylate, and ethylene glycol monosalicylate), antihistamines (such as a chemical mediator release inhibitor, histamine H1 receptor antagonists, histamine H2 receptor antagonists, histamine H3 receptor antagonists, and histamine H4 receptor antagonists), essential oil components (such as 1-menthol, camphor, limonene, isopulegol, borneol, eugenol, eucalyptus oil, peppermint oil, clove oil, cinnamon oil, and tea tree oil), antiseptics (such as isopropylmethylphenol, chlorhexidine gluconate, acrinol, and benzalkonium chloride), local anesthetics, antipruritics (such as crotamiton, ichthammol, and pine tar), blood circulation promoters (such as red pepper extract components (red pepper extract and red pepper tincture), capsaicin, dihydrocapsaicin, capsanthin, nonanoic acid vanillylamide, and nicotinic acid benzyl), steroid hormones, berberine, powdered phellodendron bark, arnica tincture, and tocopherol. The physiologically active substance may be in the form of a free body of a corresponding compound, or may be in the form of a pharmacologically acceptable salt. These second active ingredients may be used singly or in combination of two or more.

The oily component may be a component capable of constituting the oil phase of the emulsified gel composition. Examples of the oily component include vegetable oils such as avocado oil, linseed oil, olive oil, orange oil, chamomile oil, sesame oil, wheat germ oil, rice bran oil, safflower oil, squalane (phytosqualane, olive squalane or the like), squalene, soybean oil, tea oil, evening primrose oil, camellia oil, turpentine oil, corn oil, rapeseed oil, palm oil, peppermint oil, castor oil, sunflower oil, jojoba oil, cottonseed oil, coconut oil, eucalyptus oil, peanut oil, lemon oil, and rose oil, animal fats and oils such as beef tallow, squalane, squalene, turtle oil, butterfat, horse oil, mink oil, lanolin, and egg yolk oil, cholesterols (such as cholesterol and phytosterol), fatty acids (such as capric acid and oleic acid), aliphatic alcohols (such as oleyl alcohol, lauryl alcohol, and isostearyl alcohol), fatty acid esters (such as diisopropyl adipate and isopropyl palmitate), paraffin oil, and silicone oil.

The absorption enhancer may be any one having a function to enhance transdermal absorption of diclofenac or a salt thereof, and may be selected in accordance with the type of diclofenac or a salt thereof. Examples of the absorption enhancer include fatty acid esters such as diethyl sebacate and diisopropyl adipate, propylene carbonate, crotamiton, and propylene glycol.

Examples of the dissolving agent include higher alcohols (such as cetyl alcohol, stearyl alcohol, batyl alcohol, behenyl alcohol, oleyl alcohol, hexadecyl alcohol and octyldodecanol), fatty acid esters (such as isopropyl myristate, octyldodecyl myristate, cetyl myristate, myristyl myristate, diethyl sebacate, diisopropyl sebacate, diisopropyl adipate, oleyl oleate, hexyl laurate, cetyl isooctanoate, medium-chain triglyceride, and propylene glycol fatty acid ester), N-methyl-2-pyrrolidone, triacetin, benzyl alcohol, 1-menthyl glyceryl ether, polyhydric alcohols (such as glycerin, propylene glycol, polyethylene glycol, polypropylene glycol, sorbitol, 1,3-butylene glycol, dipropylene glycol, and 1-menthoxypropane-1,2-diol), and dimethyl sulfoxide.

The pH regulator is a component to be added for making the pH of the emulsified gel composition suitable to the skin. Examples of the pH regulator include an acidifying agent such as citric acid, acetic acid, lactic acid, or phosphoric acid, and an alkalizing agent such as monoethanolamine, diethanolamine, triethanolamine, or sodium hydrogen phosphate. A preferable pH regulator is diethanolamine.

The emulsified gel composition of the present embodiment has a pH of preferably 7.0 or more, more preferably more than 7.0, and further preferably 7.3 or more. When the pH is 7.3 or more, the stability of the drug (diclofenac sodium) becomes higher, and the transdermal absorption also becomes more excellent. Besides, the pH of the emulsified gel composition of the present embodiment is preferably 8 or less.

For obtaining the emulsified gel composition of the present embodiment, for example, the respective components can be weighed, and mixed by stirring or the like to obtain an emulsified composition.

In preparation of the gel composition, a hydrophobically-modified component may be added and mixed after forming the aqueous phase by mixing a water-soluble component, or a water-soluble component may be added and mixed after forming the oil phase by mixing a hydrophobically-modified component, or another method may be employed.

As a method for emulsifying the gel composition, the components may be simply mixed, or high shear force may be applied in mixing by using a high pressure homogenizer or a high-speed stirrer. Besides, the composition may be emulsified under heating or cooling. The emulsification method may be appropriately selected by those skilled in the art in consideration of a combination of a water-soluble component and a hydrophobically-modified component, and the presence/absence of an emulsifier.

### Examples

### Test 1: Antioxidant

Respective components were mixed as shown in Tables 1 and 2 to prepare compositions of Reference Examples 1 to 22. A "Component A" and a content thereof shown in Table 1 were set as shown in Table 2, and a content of purified water was set to make the whole composition 100% by mass. Additional Components shown in Table 1 refer to a physiologically active substance excluding diclofenac sodium, and another optional component.

Each of the thus obtained compositions was enclosed in a vial, and the resultant vial was allowed to stand vertically in a thermostatic chamber at 60°C to be stored therein for 1 month. After 1 month passed, the vial was taken out of the thermostatic chamber. The composition was taken out of the vial, and a diclofenac concentration therein was quantitatively determined by high performance liquid chromatography. Besides, an amount of diclofenac contained in each composition immediately after the preparation was used as an initial value (100%) to calculate a residual ratio (%) of diclofenac comprised in the composition after the storage.

The results are shown in Table 2. Evaluation results were sorted in accordance with the following criteria.
A: The residual ratio was 99% or more.
B: The residual ratio was 98% or more and less than 99%.
C: The residual ratio was 97% or more and less than 98%.
D: The residual ratio was less than 97%.

**[Table 1]**

| | Content [mass%] |
|---|---|
| Diclofenac Sodium | 1 |
| Component A | Shown in Table 2 |
| Polysorbate 80 (HLB = 15.0) | 0.1 |
| Macrogol 400 | 1 |
| Lactic Acid | 0.05 |
| Propylene Glycol | 2 |
| Diisopropyl Adipate | 5 |
| Purified Water | Balance |
| Ethanol | 45 |
| Additional Components | 3 |
| Total | 100 |

**[Table 2]**

| | Component A | Content of Component A [mass%] | Drug Residual Ratio |
|---|---|---|---|
| Reference Example 1 | None | 0 | C |
| Reference Example 2 | 2-Mercaptobenzimidazole | 0.62 | A |
| Reference Example 3 | 2-Mercaptobenzimidazole | 0.31 | A |
| Reference Example 4 | 2-Mercaptobenzimidazole | 0.06 | A |
| Reference Example 5 | Disodium Edetate | 0.8 | A |
| Reference Example 6 | Disodium Edetate | 0.01 | A |
| Reference Example 7 | Disodium Edetate | 0.005 | A |
| Reference Example 8 | Sodium Hydrogen Sulfite | 0.3 | A |
| Reference Example 9 | Sodium Hydrogen Sulfite | 0.15 | B |
| Reference Example 10 | Sodium Hydrogen Sulfite | 0.03 | D |
| Reference Example 11 | Sodium Pyrosulfite | 0.15 | B |
| Reference Example 12 | Sodium Pyrosulfite | 0.07 | D |
| Reference Example 13 | Sodium Pyrosulfite | 0.01 | D |
| Reference Example 14 | Tocopherol | 0.1 | A |
| Reference Example 15 | Tocopherol | 0.05 | A |
| Reference Example 16 | Tocopherol | 0.01 | A |
| Reference Example 17 | Dibutyl Hydroxytoluene | 1 | A |
| Reference Example 18 | Dibutyl Hydroxytoluene | 0.5 | A |
| Reference Example 19 | Dibutyl Hydroxytoluene | 0.1 | A |
| Reference Example 20 | Propyl Gallate | 0.2 | A |
| Reference Example 21 | Propyl Gallate | 0.1 | A |
| Reference Example 22 | Propyl Gallate | 0.02 | A |

### Test 2: Evaluation of Drug Stability

Respective components were mixed as shown in Table 3 to prepare emulsified gel compositions. Each numerical value shown in Table 3 is in "% by mass". Stearyloxy HPMC (stearyloxy hydroxypropyloxy group = 0.3 to 0.6% by mass) was used as hydrophobically-modified HPMC. Additional Components shown in Table 3 refer to a physiologically active substance excluding diclofenac sodium, and another optional component.

**[Table 3]**

| | Reference Example 23 | Reference Example 24 |
|---|---|---|
| Diclofenac Sodium | 1 | 1 |
| Hydrophobically-modified HPMC | 0.9 | 0.9 |
| HPC | 1 | 1 |
| Dibutyl Hydroxytoluene | 0.1 | 0.1 |
| Lactic Acid | 0 | 0 |
| Diethanolamine | 0 | 0.02 |
| Nikasol TS620 | 5 | 5 |
| Propylene Glycol | 5 | 5 |
| Diisopropyl Adipate | 7 | 7 |
| Purified Water | Balance | Balance |
| Ethanol | Balance | Balance |
| Additional Components | 7.36 | 7.36 |
| Total | 100 | 100 |
| pH | 7.3 | 8.0 |

The emulsified gel composition of each of Reference Examples 23 and 24 thus obtained was enclosed in an aluminum laminated tube, and the resultant tube was allowed to stand in a thermostatic chamber at 60°C. After 1 month passed, the tube was taken out of the thermostatic chamber. The emulsified gel composition was visually observed to measure a thickness of a separated oil layer. The emulsified gel composition was taken out of the tube, and concentrations of a decomposition product 1 (1-[2,6-dichlorophenyl]-2-indolinone) and a decomposition product 2 (ethyl 2-[(2,6-dichlorophenyl)amino]phenylacetate) were calculated by high performance liquid chromatography through conversion using a calibration curve of diclofenac. The concentrations of the respective compounds are shown in Table 4 in the form of a relative value with respect to a theoretical amount (with the diclofenac concentration at the time of preparation regarded as 100%).

**[Table 4]**

| | | Reference Example 23 | Reference Example 24 |
|---|---|---|---|
| Initial | Diclofenac | 101.28 | 101.32 |
| | Decomposition Product 1 | 0 | 0 |
| | Decomposition Product 2 | 0.05 | 0.02 |
| | Total Amount of Decomposition Products | 0.1 | 0.07 |
| After 1 Month at 60°C | Diclofenac | 101.32 | 99.41 |
| | Decomposition Product 1 | 0 | 0.54 |
| | Decomposition Product 2 | 0.02 | 0.51 |
| | Total Amount of Decomposition Products | 0.07 | 1.19 |

### Test 3: Evaluation of Storage Stability of Gel Composition

Respective components were mixed as shown in Tables 5 and 6 to prepare gel compositions. Each numerical value shown in Table 5 is in "% by mass", and a component shown in Table 6 was used as a component C. As for purified water and absolute ethanol, a mixed solution of purified water and absolute ethanol (mass ratio = 1:1) was added so that the ultimate total mass of the gel composition could be 100-fold of the mass of diclofenac sodium. Stearyloxy HPMC (stearyloxy hydroxypropyloxy group = 0.3 to 0.6% by mass) was used as hydrophobically-modified HPMC. Additional Components shown in Table 5 refer to a physiologically active substance excluding diclofenac sodium, and another optional component.

**[Table 5]**

| | Content [mass%] |
|---|---|
| Diclofenac Sodium | 1 |
| Hydrophobically-modified HPMC | 0.9 |
| HPC | 1 |
| Component B | 3 |
| Dibutyl Hydroxytoluene | 0.1 |
| 1-Methyl Glyceryl Ether | 2.5 |
| Propylene Glycol | 5 |
| Diisopropyl Adipate | 7 |
| Purified Water | Balance |
| Ethanol | Balance |
| Additional Components | 4.97 |
| Total | 100 |

**[Table 6]**

| | Component B | HLB Value |
|---|---|---|
| Comparative Example 1 | None | - |
| Comparative Example 2 | Glyceryl Monostearate | 4.0 |
| Comparative Example 3 | Propylene Glycol Monostearate | 3.5 |
| Comparative Example 4 | Decaglyceryl Pentaoleate | 3.5 |
| Comparative Example 5 | Ethylene Glycol Monostearate | 3.5 |
| Comparative Example 6 | Sorbitan Sesquioleate | 4.0 |
| Comparative Example 7 | Sorbitan Monoisostearate | 5.0 |
| Comparative Example 8 | POE (6) Sorbit Beeswax | 7.5 |
| Comparative Example 9 | POE (5) Glyceryl Oleate | 9.5 |
| Comparative Example 10 | POE (20) Sorbitan Trioleate | 11.0 |
| Example 1 | Polyethylene Glycol Monostearate (45EO) | 18.0 |
| Example 2 | Polyoxyethylene Phytosterol | 18.0 |
| Example 3 | POE (40) Cetyl Ether | 20.0 |

Each of the thus obtained gel compositions of Comparative Examples 1 to 10 and Examples 1 to 3 was enclosed in a test tube in a depth of the gel composition of 10 cm, and the resultant test tube was allowed to stand in a thermostat chamber at 60°C. After 13 days passed, the test tube was taken out of the thermostatic chamber, and the gel composition contained therein was visually observed through a side surface of the test tube to measure a thickness of a separated oil layer.

The results are shown in Table 7 and Figure 1. In the gel compositions of Comparative Examples 2 to 10, the amount of the oil layer separated during the storage at 60°C for 13 days was larger as compared with that in the gel composition of Comparative Examples 1 not comprising a surfactant, and thus, it was found that the storage stability was lowered. On the other hand, in the gel compositions of Examples 1 to 3, the oil layer was not separated after the storage, and a homogeneous state was retained.

**[Table 7]**

| | Thickness of Oil Layer [mm] |
|---|---|
| Comparative Example 1 | 1 |
| Comparative Example 2 | 4 |
| Comparative Example 3 | 29 |
| Comparative Example 4 | 25 |
| Comparative Example 5 | 27 |
| Comparative Example 6 | 24 |
| Comparative Example 7 | 19 |
| Comparative Example 8 | 4 |
| Comparative Example 9 | 5 |
| Comparative Example 10 | 8 |
| Example 1 | 0 |
| Example 2 | 0 |
| Example 3 | 0 |

### Test 4: Evaluation of Drug Stability

Respective components were mixed as shown in Table 8 to prepare emulsified gel compositions. Each numerical value shown in Table 8 is in "% by mass". The used hydrophobically-modified HPMC was the same as that described in Test 2. Additional Components shown in Table 8 refer to a physiologically active substance excluding diclofenac sodium, and another optional component. Lactic acid was added in an amount for obtaining a desired pH value of the gel composition. Polysorbate 80 has an HLB value of 15.0.

**[Table 8]**

| | Comp. Example 11 | Example 4 | Example 5 |
|---|---|---|---|
| Diclofenac Sodium | 1 | 1 | 1 |
| Hydrophobically-modified HPMC | 0.9 | 0.9 | 0.9 |
| HPC | 1 | 1 | 1 |
| 2-Mercaptobenzimid azole | - | 0.1 | - |
| Disodium Edetate | - | - | 0.1 |
| Tocopherol | - | - | - |
| Dibutyl Hydroxytoluene | - | - | - |
| Propyl Gallate | - | - | - |
| Macrogol 400 | 1 | 1 | 1 |
| Propylene Glycol | 2 | 2 | 2 |
| Polysorbate 80 | 0.1 | 0.1 | 0.1 |
| Diisopropyl Adipate | 5 | 5 | 5 |
| Purified Water | Balance | Balance | Balance |
| Ethanol | 45 | 45 | 45 |
| Lactic Acid | q. s. | q. s. | q. s. |
| Additional Components | 3 | 3 | 3 |
| Total | 100 | 100 | 100 |

| | Example 6 | Example 7 | Example 8 |
|---|---|---|---|
| Diclofenac Sodium | 1 | 1 | 1 |
| Hydrophobically-modified HPMC | 0.9 | 0.9 | 0.9 |
| HPC | 1 | 1 | 1 |
| 2-Mercaptobenzimid azole | | | |
| Disodium Edetate | - | - | - |
| Tocopherol | 0.1 | - | - |
| Dibutyl Hydroxytoluene | - | 0.1 | - |
| Propyl Gallate | - | - | 0.1 |
| Macrogol 400 | 1 | 1 | 1 |
| Propylene Glycol | 2 | 2 | 2 |
| Polysorbate 80 | 0.1 | 0.1 | 0.1 |
| Diisopropyl Adipate | 5 | 5 | 5 |

| Purified Water | Balance | Balance | Balance |
|---|---|---|---|
| Ethanol | 45 | 45 | 45 |
| Lactic Acid | q. s. | q. s. | q. s. |
| Additional Components | 3 | 3 | 3 |
| Total | 100 | 100 | 100 |

Each of the thus obtained emulsified gel compositions was filled in an aluminum laminated tube, and the resultant tube was stored in a thermostatic chamber at 60°C for 1 month. After 1 month, the aluminum laminated tube was taken out of the thermostatic chamber. The composition was taken out of the aluminum laminated tube, and a residual amount of diclofenac was calculated by high performance liquid chromatography. A diclofenac concentration thus obtained was shown in Table 9 as a relative value to a theoretical amount (with the diclofenac concentration at the time of preparation regarded as 100%). The thus obtained value (drug residual ratio) was sorted in accordance with the evaluation criteria of Test 1.

The results are shown in Table 9. The drug residual ratio was higher in Examples 4 to 8 than in Comparative Example 11.

**[Table 9]**

| | Comp. Example 11 | Example 4 | Example 5 |
|---|---|---|---|
| pH | 7.0 | 7.1 | 7.1 |
| Drug Residual Ratio [%] | 97.0 | 98.5 | 98.1 |
| Drug Residual Ratio | C | B | B |

| | Example 6 | Example 7 | Example 8 |
|---|---|---|---|
| pH | 7.0 | 7.0 | 7.1 |
| Drug Residual Ratio [%] | 98.3 | 98.3 | 98.2 |
| Drug Residual Ratio | B | B | B |

### Test 5: Evaluation of Drug Stability

Respective components were mixed as shown in Table 10 to prepare emulsified gel compositions. Each numerical value shown in Table 10 is in "% by mass". The used hydrophobically-modified HPMC was the same as that described in Test 2. Additional Components shown in Table 10 refer to a physiologically active substance excluding diclofenac sodium, and another optional component. Lactic acid and diethanolamine were added in amounts for obtaining a desired pH value of the gel composition. POE (30) behenyl ether has an HLB value of 18.0.

**[Table 10]**

| | Example 9 | Example 10 | Example 11 |
|---|---|---|---|
| Diclofenac Sodium | 1 | 1 | 1 |
| Hydrophobically-modified HPMC | 0.9 | 0.9 | 0.9 |
| HPC | 1 | 1 | 1 |
| Dibutyl Hydroxytoluene | 0.1 | 0.1 | 0.1 |
| Lactic Acid | 0.002 | - | - |
| Diethanolamine | - | 0.017 | 0.068 |
| Nikasol TS620 | 5 | 5 | 5 |
| Purified Water | Balance | Balance | Balance |
| Ethanol | Balance | Balance | Balance |
| Propylene Glycol | 5 | 5 | 5 |
| Diisopropyl Adipate | 7 | 7 | 7 |
| POE (30) Behenyl Ether | 2 | 2 | 2 |
| Additional Components | 7.36 | 7.36 | 7.36 |
| Total | 100 | 100 | 100 |

Each of the thus obtained emulsified gel compositions was filled in an aluminum laminated tube, and the resultant tube was stored in a thermostatic chamber at 60°C for 1 month. After 1 month, the aluminum laminated tube was taken out of the thermostatic chamber. The composition was taken out of the aluminum laminated tube, and a residual amount of diclofenac was calculated by high speed performance chromatography. A diclofenac concentration thus obtained was shown in Table 11 as a relative value to a theoretical amount (with the diclofenac concentration at the time of preparation regarded as 100%). The thus obtained value (drug residual ratio) was sorted in accordance with the evaluation criteria of Test 1.

Results are shown in Table 11. The drug residual ratio was high in all of Examples 9 to 11.

**[Table 11]**

| | Example 9 | Example 10 | Example 11 |
|---|---|---|---|
| pH | 7.0 | 7.3 | 8.0 |
| Drug Residual Ratio [%] | 98.3 | 98.0 | 98.4 |
| Drug Residual Ratio | B | B | B |

## Claims

1. An emulsified gel composition comprising diclofenac sodium, water, a gelling agent, an antioxidant, and a surfactant having an HLB of 14 or more,
wherein the gelling agent is a nonionic water-soluble polymer selected from the group consisting of hydroxypropyl cellulose and hydrophobically-modified hydroxypropyl methyl cellulose, and
wherein the composition does not comprise an ionic polymer or comprises an ionic polymer in an amount of 0.5% by mass or less based on the mass of the whole emulsified gel composition.

2. The emulsified gel composition according to claim 1, wherein the nonionic water-soluble polymer comprises hydrophobically-modified hydroxypropyl methyl cellulose.

3. The emulsified gel composition according to claim 1 or 2, wherein the antioxidant comprises at least one compound selected from the group consisting of 2-mercaptobenzimidazole, disodium edetate, tocopherol, dibutyl hydroxytoluene, and propyl gallate.

4. The emulsified gel composition according to any one of claims 1 to 3, wherein the surfactant having an HLB of 14 or more comprises at least one compound selected from the group consisting of polyoxyethylene sorbitan monococonut fatty acid ester, polyethylene glycol monostearate, polyoxyethylene phytosterol, polyoxyethylene cetyl ether, polyoxyethylene behenyl ether, and polyoxyethylene oleyl ether.

5. The emulsified gel composition according to any one of claims 1 to 4, wherein a pH of the emulsified gel composition is 7.0 or more.

## Patentansprüche

1. Emulgierte Gelzusammensetzung, umfassend Diclofenac-Natrium, Wasser, ein Geliermittel, ein Antioxidationsmittel und ein Tensid mit einem HLB-Wert von 14 oder mehr,
wobei das Geliermittel ein nichtionisches, wasserlösliches Polymer ist, ausgewählt aus der Gruppe bestehend aus Hydroxypropylcellulose und hydrophob-modifizierter Hydroxypropylmethylcellulose, und
wobei die Zusammensetzung kein ionisches Polymer oder ein ionisches Polymer in einer Menge von 0,5 Massen% oder weniger, bezogen auf die Masse der gesamten emulgierten Gelzusammensetzung, enthält.

2. Emulgierte Gelzusammensetzung nach Anspruch 1, wobei das nichtionische wasserlösliche Polymer hydrophob-modifizierte Hydroxypropylmethylcellulose umfasst.

3. Emulgierte Gelzusammensetzung nach Anspruch 1 oder 2, wobei das Antioxidationsmittel mindestens eine Verbindung, ausgewählt aus der Gruppe bestehend aus 2-Mercaptobenzimidazol, Dinatriumedetat, Tocopherol, Dibutylhydroxytoluol und Propylgallat, umfasst.

4. Emulgierte Gelzusammensetzung nach einem der Ansprüche 1 bis 3, wobei das Tensid mit einem HLB-Wert von 14 oder mehr mindestens eine Verbindung, ausgewählt aus der Gruppe bestehend aus Polyoxyethylensorbitanmonokokosnussfettsäureester, Polyethylenglycolmonostearat, Polyoxyethylenphytosterol, Polyoxyethylencetylether, Polyoxyethylenbehenylether und Polyoxyethylenoleylether, umfasst.

5. Emulgierte Gelzusammensetzung nach einem der Ansprüche 1 bis 4, wobei ein pH-Wert der emulgierten Gelzusammensetzung 7,0 oder mehr beträgt.

## Revendications

1. Composition de gel émulsifié comprenant du diclofénac sodique, de l'eau, un agent gélifiant, un antioxydant et un surfactant ayant un HLB de 14 ou plus,
dans laquelle l'agent gélifiant est un polymère hydrosoluble non ionique choisi dans le groupe constitué de l'hydroxypropylcellulose et de l'hydroxypropylméthyl-cellulose modifiée par hydrophobie, et
dans laquelle la composition ne comprend pas de polymère ionique ou comprend un polymère ionique en quantité égale ou inférieure à 0,5 % en masse par rapport à la masse de l'ensemble de la composition de gel émulsifié.

2. Composition de gel émulsifié selon la revendication 1, dans laquelle le polymère hydrosoluble non ionique comprend de l'hydroxypropylméthylcellulose modifiée de manière hydrophobe.

3. Composition de gel émulsifiée selon les revendications 1 ou 2, dans laquelle l'antioxydant comprend au moins un composé choisi dans le groupe constitué de 2-mercaptobenzimidazole, d'édétate disodique, de tocophérol, de dibutylhydroxytoluène et de gallate de propyle.

4. Composition de gel émulsifié selon l'une des revendications 1 à 3, dans laquelle le tensioactif ayant un HLB de 14 ou plus comprend au moins un composé choisi dans le groupe constitué de l'ester d'acide gras de polyoxyéthylène sorbitan monococonut, du monostéarate de polyéthylène glycol, du phytostérol de polyoxyéthylène, de l'éther de polyoxyéthylène cétyle, de l'éther de polyoxyéthylène béhényle, et de l'éther de polyoxyéthylène oléyle.

5. Composition de gel émulsifiée selon l'une des revendications 1 à 4, dans laquelle le pH de la composition de gel émulsifiée est égal ou supérieur à 7,0.
